# EUROPÄISCHE PATENTSCHRIFT

(11) **EP 3 034 107 B1**
(45) Veröffentlichungstag und Bekanntmachung des Hinweises auf die Patenterteilung: **14.02.2018**
(21) Anmeldenummer: 15199476.1
(22) Anmeldetag: 11.12.2015
(51) Int. Cl.: A61M 1/14, H05K 5/02

(54) **MULTIFUNKTIONALER GRIFF EINER BLUTBEHANDLUNGSMASCHINE ZUM HÄNDISCHEN BEWEGEN DER BLUTBEHANDLUNGSMASCHINE SOWIE ZUM AUFWICKELN VON LEITUNGEN UND BLUTBEHANDLUNGSMASCHINE MIT EINEM SOLCHEN GRIFF**
MULTIFUNCTIONAL HANDLE OF A BLOOD TREATMENT MACHINE FOR MANUALLY MOVING THE BLOOD TREATMENT MACHINE AND WINDING LINES AND BLOOD TREATMENT MACHINE WITH SUCH A HANDLE
POIGNÉE MULTIFONCTIONNELLE D'UNE MACHINE DE TRAITEMENT DU SANG DESTINÉE À DÉPLACER MANUELLEMENT LA MACHINE DE TRAITEMENT DU SANG ET À ENROULER LES TUYAUX ET MACHINE DE TRAITEMENT DU SANG DOTÉE D'UNE TELLE POIGNÉE

(30) Priorität: 18.12.2014 DE 102014119107
(43) Veröffentlichungstag der Anmeldung: 22.06.2016
(73) Patentinhaber: B. Braun Avitum AG, 34212 Melsungen (DE)
(72) Erfinder: Iske, Andreas, 34320 Söhrewald (DE); Stenzel, Bruno, 34346 Hann. Münden (DE)
(74) Vertreter: Winter, Brandl, Fürniss, Hübner, Röss, Kaiser, Polte - Partnerschaft mbB

(56) Entgegenhaltungen:
- EP-A1- 0 240 153
- US-A1- 2009 012 458
- US-A1- 2009 084 717
- US-A1- 2010 089 806
- US-A1- 2011 040 242

## Beschreibung

Die vorliegende Erfindung betrifft einen multifunktionalen Griff einer mobilen Maschine zur extrakorporalen Blutbehandlung bzw. einer mobilen Blutbehandlungsmaschine zum händischen Bewegen der Blutbehandlungsmaschine sowie zum Aufwickeln von Leitungen und eine Blutbehandlungsmaschine mit einem solchen multifunktionalen Griff.

### Hintergrund der Erfindung

Die moderne extrakorporale Blutbehandlung, beispielsweise die Dialyse, wird immer komplexer und flexibler. Besonders durch eine stetig zunehmende Anzahl technischer Neuerungen und zusätzlicher Behandlungsgeräte konnte der Standard klinischer Blutbehandlungen in den letzten Jahren kontinuierlich gehoben werden. Allerdings erhöht sich durch die zunehmende Anzahl neuer technischer Geräte auch der Druck, bestehenden Bauraum bei Blutbehandlungsmaschine besser zu nutzen, um mehr und mehr technische Funktionen in einer vorzugsweise mobilen Blutbehandlungsmaschine herkömmlicher Größe unterbringen zu können. Neben der Miniaturisierung von Bauteilen und anderen technischen Bestandteilen der Blutbehandlungsmaschine spielt hierbei vor Allem das Konzipieren multifunktionaler Bauteile eine Schlüsselrolle.

### Stand der Technik

Eine herkömmliche Blutbehandlungsmaschine ist beispielsweise aus der WO 2004/028594A1 bekannt. Eine derartige mobile Blutbehandlungsmaschine weist an ihrer Außenseite mehrere separate Bauteile auf, um die Dialysemaschine händisch zu bewegen (zwei Griffe), um Fluidleitungen oder Kabel aufzuwickeln bzw. zu verstauen (eine Klemme) und, um die verschiedenen elektrischen bzw. IT-Schnittstellen der Dialysemaschine vor Spritzwasser zu schützen (ein erhöhter Rand an der tablettartig ausgebildeten Oberseite der Dialysemaschinen bzw. Abdeckungsklappen für die Schnittstellen).

Ein Wagen für ein Behandlungssystem ist beispielsweise aus der Druckschrift EP 0240153 A1 bekannt. Dieser Wagen weist eine Griffstange auf, neben der mindestens ein Haken angeordnet ist.

Zudem offenbart die Druckschrift US 2009/0012458A1 eine Dialysemaschine mit einem Griff in Form einer gebogenen Griffstange.

Bei der Blutbehandlungsmaschine gemäß der US 2010/0089806 A1 ist die Griffstange als den Korpus der Blutbehandlungsmaschine kreisförmig umlaufender Handlauf ausgestaltet. Eine sehr ähnliche Ausgestaltung ist auch in der Druckschrift US 2009/0084717A1 offenbart.

Weiterhin offenbart die Druckschrift US 2011/0040242A1 einen Wagen für eine Dialysemaschine, bei welchem der Griff als Aussparung in der Tischplatte ausgestaltet ist.

Häufig weisen herkömmliche Blutbehandlungsmaschine insbesondere der mobilen Bauart noch zusätzlich Begrenzungselemente auf, die von dem Körper der Blutbehandlungsmaschine hervorragen und einer Beschädigung des Körpers bzw. des Gehäuses der Dialysemaschine aufgrund von Kollisionen mit z.B. Wänden während des Rangierens der Dialysemaschine vorbeugen sollen.

Diese vielen separaten Bauteile beanspruchen bei der Konstruktion einer solchen Blutbehandlungsmaschine viel Bauraum, der daher nicht für unmittelbar dialyserelevante Bauteile verwendet werden kann. Aufgrund des beschränkten Bauraums können die verschiedenen Bauteile wie beispielsweise die Klemme zum Aufwickeln von Leitungen nur suboptimal positioniert werden, so dass oftmals auf die Klemme aufgewickelte Leitungen elektrische Schnittstellen und IT-Schnittstellen verdecken oder eine Bedienung der Dialysemaschine behindern.Zudem ist der Montageaufwand bei der Herstellung solcher Dialysemaschinen unnötig hoch, wodurch auch die Herstellungskosten derartiger Dialysemaschinen relativ hoch sind.

### Kurzbeschreibung der Erfindung

Ausgehend von dem vorstehend beschriebenen Stand der Technik liegt der Erfindung die Aufgabe zugrunde, eine Blutbehandlungsmaschine, vorzugsweise Dialysemaschine der mobilen Bauart bereitzustellen, die sich durch eine effizientere Bauraumnutzung, geringere Herstellungskosten und geringeren Montageaufwand auszeichnet. Insbesondere die Aufgaben des Bewegens bzw. Rangierens der vorzugsweise mobilen Blutbehandlungsmaschine und der Aufbewahrung bzw. des Aufwickelns von mit der Blutbehandlungsmaschine verbundenen Leitungen bzw. Schläuchen, aber vorzugsweise auch die Aufgaben der Begrenzung der Blutbehandlungsmaschine bzw. des Schutzes der Blutbehandlungsmaschine bei möglichen Kollisionen und der Schutz verschiedener elektrischer Schnittstellen und IT-Schnittstellen der Blutbehandlungsmaschine vor Spritzwasser sollen durch einen einzigen multifunktionalen Griff erfüllt werden.

Diese Aufgabe wird gelöst durch einen multifunktionalen Griff mit den Merkmalen gemäß dem Anspruch 1 und durch eine Blutbehandlungsmaschine, vorzugsweise Blutbehandlungsmaschine gemäß Anspruch 14 mit einem solchen multifunktionalen Griff. Vorteilhafte Weiterbildungen der Erfindung sind Gegenstand der Unteransprüche.

Der Kerngedanke der vorliegenden Erfindung besteht demzufolge darin, mehrere Funktionen bzw. Aufgaben in einem einzigen multifunktionalen Bauteil insbesondere einem Multifunktionshandgriff zu vereinen. Der erfindungsgemäße Multifunktionshandgriff hat demzufolge wenigstens zwei (bauliche) Funktionsabschnitte, von denen der Eine für das Aufnehmen/Tragen von Leitungen/Schläuchen und der Andere für das ergonomische Ergreifen durch eine Bedienperson konstruktiv angepasst sind. Dabei sind die beiden Funktionsabschnitte erfindungsgemäß nicht adaptiv sondern integrativ miteinander verknüpft, derart, dass beide Funktionsabschnitte gleichzeitig auch zur Funktionserfüllung des jeweils anderen Funktionsabschnitts beitragen.

In anderen Worten ausgedrückt hat ein Handgriff in der Regel einen mehr oder weniger ergonomisch geformten Greifabschnitt, der durch einen Abstandshalter von einer Gehäusewand oder dergleichen Bauteil beabstandet sein muss, um umfänglich stabil ergriffen werden zu können. Gemäß einem ersten Aspekt der Erfindung ist dieser Abstandshalter nunmehr zu einem Leitungs-/Schlauchträger (Aufnahme-/ Einhängabschnitt für Leitungen/Schläuche) geformt, wohingegen der Greifabschnitt gleichzeitig ein Rückhalte-/Sperrelement bildet, das ein Abgleiten/Abfallen der/des am Leitungs-/Schlauchträger aufgelagerten Leitung/Schlauchs über dessen freies Ende verhindert. Zusätzlich kann der Greifabschnitt so geformt/angeordnet sein, dass er entsprechend einer Stoßstange die Gehäusewand oder dergleichen Bauteil zumindest abschnittsweise umrahmt und somit einen Kollisionsschutz bildet.

Gemäß dem Anspruch 1 wird folglich ein multifunktionaler Griff einer mobilen Blutbehandlungsmaschine, vorzugsweise (Hämo-) Dialysemaschine, zum händischen Bewegen der Blutbehandlungsmaschine sowie zum Aufwickeln oder Einhängen von Leitungen und/oder Schläuchen vorgeschlagen, der einen bügel- oder rinnenartigen Leitungsaufnahmeabschnitt und einen ergonomisch geformten Griffabschnitt hat, der an wenigstens einem Endbereich des bügel- oder rinnenartigen Leitungsaufnahmeabschnitts angeordnet oder ausgebildet ist. Dies bedeutet, dass der multifunktionale Griff (oder Multifunktionsgriff) neben der Bereitstellung einer ergonomisch geformten Greifgeometrie gleichzeitig eine für ein Aufhängen von biegeflexiblen Leitungen optimierte Halterung bildet, welche wiederum die Greifgeometrie von der Blutbehandlungsmaschine bzw. deren Gehäuse so beabstandet, dass sie von einer Bedienperson auch umgriffen werden kann. Durch diese integrative Gestaltung des Multifunktionsgriffs sind adaptive Einrichtungen wie zusätzliche Haken, die am Griff montiert werden oder zusätzliche, beispielsweise zapfenförmige Vorsprünge am Griff als Aufhängeinrichtungen überflüssig. Auch sind die entsprechende Formgebung des Leitungsaufnahmeabschnitts sowie die Positionierung des Griffabschnitts entscheidend dafür, dass bereits eingehängte Leitungen/Schläuche nicht unbeabsichtigt abgestreift werden können.

Gemäß einer besonders bevorzugten Ausführungsform gemäß Anspruch 2 weist der Multifunktionshandgriff einen Abrutschverhinderungsabschnitt auf, der zwischen dem bügel-oder rinnenartigen Leitungsaufnahmeabschnitt und dem ergonomisch geformten Griffabschnitt angeordnet und hin zu der mobilen Blutbehandlungsmaschine derart gewölbt ist, dass ein Abrutschen von in dem bügel-oder rinnenartigen Leitungsaufnahmeabschnitt aufgenommenen Leitungen und / oder Schläuchen verhindert wird. Diese Ausgestaltung des Multifunktionshandgriffs ermöglicht ein besonders einfaches und sicheres Verwahren von Schläuchen auf dem Handgriff. Ein derartiger erfindungsgemäßer Multifunktionshandgriff kann daher nicht nur lediglich zweckfremd verwendet werden, sondern ist speziell für diese zweite Funktion ausgelegt, ohne dabei Funktionseinbußen bei der Erfüllung der Hauptfunktion zu erleiden.

Eine bevorzugte Weiterbildung der Erfindung sieht vor, dass der Griffabschnitt handlauf- oder stangenartig ausgeformt ist und sich längs zu einer durch den Leitungsaufnahmeabschnitt definierten Leitungsaufnahmerichtung erstreckt. Dadurch kann der Griff beispielsweise auf Schulterbreite erstreckt werden, um so genügend manuelle Kraft auf die Blutbehandlungsmaschine zu deren Bewegung übertragen zu können.

Vorzugsweise kann es vorgesehen sein, dass der Griffabschnitt an wenigstens einem quer zur Leitungsaufnahmerichtung sich befindlichen Ende (Stirnseite) des Leitungsaufnahmeabschnitts positioniert ist. In diesem Fall ist also der Griff vorzugsweise in zwei Griffteile zerlegt, die an den beiden in Leitungsaufnahmerichtung gesehenen Stirnseiten des Leitungsaufnahmeabschnitts angeordnet sind und diesen zwischen sich einschließen. Alternativ hierzu kann es aber auch vorgesehen sein, dass der Griffabschnitt an einem längs zur Leitungsaufnahmerichtung sich befindenden Ende des Leitungsaufnahmeabschnitts positioniert ist. Dieses Ende kann im Fall einer Bügelart quasi punktförmig und im Fall einer Rinnenart kantenförmig sein.

Gemäß einem anderen, ggf. unabhängigen Aspekt der vorliegenden Erfindung kann es vorgesehen sein, dass der Leitungsaufnahmeabschnitt des multifunktionalen Griffs an seinem dem Griffabschnitt gegenüberliegenden Anschlussende fest mit der mobilen Blutbehandlungsmaschine (Hämodialysemaschine), vorzugsweise über ein Verbindungsstück oder Montageadapter, verbunden oder verbindbar ist. Dadurch wird eine eingehängte Leitung/Schlauch zwischen dem Griffabschnitt und der Blutbehandlungsmaschine aufgelagert und damit vor einem Abrutschen gesichert. Vorzugsweise dient dabei der Leitungsaufnahmeabschnitt des multifunktionalen Griffs als Abstandshalter des Griffabschnitts zur Blutbehandlungsmaschine.

Des Weiteren kann es vorgesehen sein, dass der vorzugsweise handlauf- oder stangenartige Griffabschnitt an mindestens einem und vorzugsweise beiden seiner Längsenden in Leitungsaufnahmerichtung gesehen über den Leitungsaufnahmeabschnitt vorragt. Somit übernimmt der Griffabschnitt nicht nur die Funktion, manuelle Kraft einer Bedienperson auf die Maschine zu übertragen sondern auch die Maschine/das Maschinengehäuse vor Aufprallschäden zu schützen.

Vorteilhaft kann es sein, wenn der Leitungsaufnahmeabschnitt in Leitungsaufnahmerichtung gesehen schalenförmig gewölbt ausgebildet ist, um auf diese Weise ein Abknicken der eingehängten Leitungen und/oder Schläuche zu vermeiden. Vorzugsweise ist der Leitungsaufnahmeabschnitt als flächiges, vorzugsweise plattenartiges Bauteil ausgebildet. Er bietet somit eine glatte Auflagefläche, wodurch Beschädigungen an den Leitungen/Schläuchen verhinderbar sind.

Zur einfachen und kostengünstigen Herstellung kann der multifunktionale Griff vorzugsweise mit einem Verbindungsstück oder Montageadapter stoffeinstückig aus Metall oder Kunststoff gefertigt sein.

Schließlich betrifft ein anderer Aspekt der vorliegenden Erfindung die Bereitstellung einer Blutbehandlungsmaschine vorzugsweise mit einem Maschinengehäuse an der/dem ein multifunktionaler Griff gemäß der Erfindung montiert ist.

Aus der vorstehenden Beschreibung der Erfindung ist ersichtlich, dass der multifunktionale Griff infolge seiner Bügel- oder Rinnenbauart den Schlauchaufnahmeabschnitt bzw. Auflageabschnitt, um welchen Leitungen zur Verwahrung gewickelt werden können, und vorzugsweise einen mit dem Auflageabschnitt verbundenen Abrutschverhinderungsbereich ausbildet, der von dem Auflageabschnitt an dessen freien Endbereich in einem Winkel vorragt und somit ein Abrutschen von auf dem Auflageabschnitt verwahrten Leitungen über dessen freien Endbereich verhindert. Die Besonderheit des multifunktionalen Griffs gemäß der Erfindung liegt darin, dass der Abrutschverhinderungsabschnitt durch eine zumindest partiell ergonomische Ausgestaltung für das Ergreifen durch eine Hand ausgelegt ist, wodurch die Funktionen "leichtes/ergonomisches Ergreifen" und "Verhindern des Abrutschens aufgewickelter Leitungen" in einem baulichen Abschnitt des multifunktionalen Griffs vereint werden. Zusätzlich kann beispielsweise der Auflageabschnitt des multifunktionalen Griffs als von dem Körper/Bauteil bzw. dem Gehäuse der Blutbehandlungsmaschine (Hämodialysemaschine) vorragender, vorzugsweise flächiger Abschnitt ausgebildet sein, der zudem nach Art eines hervorstehenden (Wölb-) Daches an dem Gehäuse der Maschine angeordnete elektrische Anschlüsse und/oder IT-Schnittstellen von Spitzwasser abschirmt. Somit lassen sich auch die Funktionen " Verwahrung von Leitungen" und "Schutz vor Spritzwasser" in einem baulichen Abschnitt des multifunktionalen Griffs vereinen.

Der Ausgestaltung des multifunktionalen Griffs gemäß Anspruch 1 liegt somit das Konstruktionskonzept zugrunde, jeden baulichen Abschnitt des Griffs möglichst so zu konzipieren, dass dieser eine bauliche Abschnitt für die Erfüllung möglichst vieler verschiedener Funktionen angepasst ist und somit die Funktionalität des gesamten Multifunktionsgriffs auf einen möglichst weiten Aufgabenbereich ausgedehnt werden kann.

Durch den multifunktionalen Griff gemäß der vorliegenden Erfindung lassen sich der Montageaufwand und die Herstellungskosten einer vorzugsweise mobilen Blutbehandlungsmaschinen wie einer Hämodialysemaschine reduzieren, da anstatt mehrerer separater Bauteile zur Erfüllung der Aufgaben "Rangieren", "Aufbewahren", "Begrenzen" und "Schutz empfindlicher Schnittstellen gegen Spritzwasser" nur ein einziges Bauteil, nämlich der multifunktionale Griff gemäß der Erfindung, montiert werden muss. Durch die effizientere Bauraumnutzung erhöht sich auch die Flexibilität bei der technischen Ausgestaltung der Blutbehandlungsmaschine (Hämodialysemaschine). So muss beispielsweise die tablettartig ausgestaltete Oberseite der Maschine keinen hohen Rand mehr aufweisen, der ein Greifen von auf der Oberseite abgestellten Gegenständen behindern kann, da die Abschirmung von empfindlichen elektrischen und IT- Schnittstellen der Maschine bereits durch den multifunktionalen Griff erfolgt.

Abschließend sei darauf hingewiesen, dass der Schlauchaufnahmeabschnitt/Auflageabschnitt nicht notwendiger Weise als Fläche (Platte) ausgebildet sein muss, sondern auch aus mindestens einer Stange bestehen kann, von der ein Abrutschverhinderungsabschnitt in Form einer zweiten Stange in einem definierten Winkel aufragt und dadurch die Bügelart definiert. Zum verbesserten Greifen kann der Abrutschverhinderungsabschnitt beispielsweise an dem dem Auflageabschnitt abgewandten (freien) Ende der Stange eine Griffkugel oder eine Griffstange bzw. einen Handlauf aufweisen. Weiterhin alternativ können der Auflageabschnitt und der Abrutschverhinderungsabschnitt des multifunktionalen Griffs in einer einzigen gebogenen Stange oder Platte (Hakenform) baulich integriert sein. In diesem Falle bestimmt die Biegung der Stange den Winkel zwischen dem Auflageabschnitt und dem Abrutschverhinderungsabschnitt des multifunktionalen Griffs.

In einer vorteilhaften Ausführungsform ist der Winkel zwischen dem Auflageabschnitt und dem Abrutschverhinderungsabschnitt im Wesentlichen rechtwinklig. Hierdurch wird ein im Wesentlichen L-förmiger Griff (im Querschnitt) gebildet.

Vorteilhafterweise ragt bei dieser Anordnung der Auflageabschnitt des multifunktionalen Griffs rechtwinklig von dem Körper bzw. dem Gehäuse der Blutbehandlungsmaschine vor und der Abrutschverhinderungsabschnitt ragt im Wesentlichen rechtwinklig von dem Auslageabschnitt hin zu der Oberseite der Blutbehandlungsmaschine vor, sodass zwischen dem Abrutschverhinderungsabschnitt und der Oberfläche des Körpers bzw. Gehäuses der Blutbehandlungsmaschine quasi eine Mulde/Rinne gebildet wird. Wird der ergonomisch ausgeformte Abrutschverhinderungsabschnitt zum Rangieren der mobilen Blutbehandlungsmaschine ergriffen, so befindet sich die Mulde/Rinne von der den multifunktionalen Griff ergreifenden Bedienperson aus gesehen hinter dem Abrutschverhinderungsabschnitt. Da die Mulde/Rinne der Aufnahme von Leitungen dient, ist diese Anordnung besonders vorteilhaft, da in der Mulde/Rinne liegende, um den Auflageabschnitt des multifunktionalen Griffs aufgewickelte Leitungen bei dieser Anordnung ein Ergreifen des Abrutschverhinderungsabschnitts des multifunktionalen Griffs nicht behindern.

Alternativ kann der Abrutschverhinderungsabschnitt des multifunktionalen Griffs an seiner dem Auflageabschnitt abgewandten Seite fest mit der Oberfläche des Körpers bzw. des Gehäuses der Blutbehandlungsmaschine, vorzugsweise über ein Verbindungsstück, verbunden sein. Bei einer derartigen Rücken- an- Rücken- Verbindung zwischen dem multifunktionalen Griff und dem Körper der Blutbehandlungsmaschine bildet der Auflageabschnitt des multifunktionalen Griffs eine Auflagefläche für aufgewickelte Kabel, die von einer den multifunktionalen Griff ergreifenden Bedienperson aus gesehen, vor dem Abrutschverhinderungsabschnitt liegt. Um ein Abrutschen von auf der Auflagefläche liegenden, um den Auflageabschnitt des multifunktionalen Griffs aufgewickelten Leitungen zu verhindern, kann in dieser Ausführungsform das dem Abrutschverhinderungsabschnitt abgewandte Ende des Auflageabschnitts nach oben, d.h. in Richtung hin zu der Oberseite der Blutbehandlungsmaschine gebogen sein und somit als zweiter, zusätzlicher Abrutschverhinderungsabschnitt dienen. In diesem Fall würde der Griff im Querschnitt quasi eine U-Form annähern.

Ein Vorteil der geschilderten Anordnungen des multifunktionalen Griffs an dem Körper einer Blutbehandlungsmaschine besteht darin, dass der Auflageabschnitt von dem Körper bzw. dem Gehäuse der Blutbehandlungsmaschine im Wesentlichen rechtwinklig vorsteht. Werden empfindliche elektrische Schnittstellen oder IT-Schnittstellen am Körper der Blutbehandlungsmaschine von der Oberseite der Blutbehandlungsmaschine betrachtet unter dem Auflageabschnitt des multifunktionalen Griffs angeordnet, so schirmt der vorstehende Auflageabschnitt die elektrischen bzw. IT-Schnittstellen nach Art eines hervorstehenden Daches vor von oben herabfallendem Spritzwasser ab. Die Notwendigkeit separater Abdeckungen für diese Schnittstellen entfällt daher durch die Ausgestaltung des multifunktionalen Griffs.

Da der multifunktionale Griff zudem als Begrenzungselement dient, d. h. ähnlich zu einer Stoßstange die Blutbehandlungsmaschine bei Kollisionen schützt, entfällt zudem die Notwendigkeit eines separaten Begrenzungselements.

Weiterhin wird gemäß einer vorteilhaften Abwandlung des multifunktionalen Griffs das Rangieren der mobilen Blutbehandlungsmaschine dadurch erleichtert, dass die Griffstange an mindestens einem ihrer Enden über den Auflageabschnitt in Auflagerichtung gesehen vorragt. Besonders vorteilhaft ist es, wenn die Griffstange bzw. der Handlauf an ihren bzw. seinen beiden Enden um eine gleiche Länge über den Auflageabschnitt vorragt, da diese Anordnung sowohl ein Rangieren der mobilen Blutbehandlungsmaschine nach links als auch nach rechts erleichtert.

Gemäß einer weiteren vorteilhaften Ausgestaltung ist mindestens einer von dem Auflageabschnitt und dem Abrutschverhinderungsabschnitt des multifunktionalen Griffs gekrümmt ausgebildet. Beispielsweise ist der Auflageabschnitt, der die Mulde/Rinne bzw. Auflagefläche zur Aufnahme von Leitungen bildet, als Mantelfläche eines Halbzylinders ausgebildet. Eine derartige Ausgestaltung verhindert eine Herabrutschen bzw. Herausfallen der Leitungen von der Auflagefläche bzw. der Mulde/Rinne. Der von dem Auflageabschnitt aufragende Abrutschverhinderungsabschnitt kann derart gewölbt sein, dass er eine beispielsweise konvexe Wölbung der Oberfläche des Körpers der Blutbehandlungsmaschine nachbildet. Zusätzlich oder alternativ kann auch der Abrutschverhinderungsabschnitt als eine Mantelfläche eines Halbzylinders ausgebildet sein, der hin zu der Oberfläche des Körpers bzw. des Gehäuses der Blutbehandlungsmaschine konvex gewölbt ist. Bei einer derartigen Ausgestaltung werden zwischen dem Abrutschverhinderungsabschnitt und der Oberfläche des Körpers der Blutbehandlungsmaschine aufbewahrte Leitungen durch die Wölbung des Abrutschverhinderungsabschnitt eingeklemmt, wodurch ein Herausfallen der Leitungen aus der Lagerposition verhindert wird.

Zudem bietet die Reduktion der Anzahl der an der Blutbehandlungsmaschine befindlichen Kleinteile auch hygienische Vorteile: Durch den multifunktionalen Griff entstehen große, ebene, abgerundete und spaltfreie Flächen, die einfach zu reinigen sind. Dies ist insbesondere im hektischen klinischen Alltag essentiell, da Oberflächen häufig gereinigt werden müssen, hierfür aber oftmals nicht viel Zeit zur Verfügung steht.

Zur weiteren Verbesserung der Effizienz der Bauraumnutzung der Blutbehandlungsmaschine kann der multifunktionale Griff auch als Plattform zur Integration von weiteren für die Dialyse notwendigen Bauteilen und Schnittstellen genutzt werden. So können beispielsweise Belüftungsvorrichtungen oder IT-Schnittstellen bzw. Anschlüsse in dem multifunktionalen Griff integriert werden. Beispielsweise kann der multifunktionale Griff entlang seiner Länge (In Aufnahmerichtung gesehen) Schienen aufweisen, die der Führung eines Lüftervlieses dienen. So erhöhen sich die Möglichkeiten, die Belüftungsvorrichtung möglichst vorteilhaft anzuordnen und den zur Verfügung stehenden Bauraum optimal zu nutzen. Auch verschiedene Anschlüsse, beispielsweise zum Anschließen verschiedener Kabel, können vorzugsweise an den seitlichen Enden oder der Unterseite des multifunktionalen Griffs angeordnet sein.

Zudem kann der multifunktionale Griff auch eine integrierte Medienleiste, welche vorzugsweise unter dem Leitungsaufnahmeabschnitt an dem Körper der Blutbehandlungsmaschine angeordnet ist, aufweisen. Durch diese Anordnung schirmt der Leitungsaufnahmeabschnitt des multifunktionalen Griffs die verschiedenen Anschlüsse und Schnittstellen von von oben herabfallendem Spritzwasser ab. Vorteilhafterweise ist der multifunktionale Griff hierbei so ausgebildet, dass die verschiedenen Anschlüsse auch dann zugänglich sind, wenn Leitungen auf dem Leitungsaufnahmeabschnitt aufgewickelt sind.

Gemäß einer vorteilhaften Ausgestaltung ist der Leitungsaufnahmeabschnitt derart ausgebildet, dass er an seiner Unterseite einen Hohlraum bildet. Beispielsweise ist der Leitungsaufnahmeabschnitt gekrümmt bzw. als konvex nach oben gewölbter Ausschnitt einer Zylindermantelfläche ausgebildet. Durch diese geometrische Ausgestaltung des Leitungsaufnahmeabschnitts wird ein von drei Seiten durch den multifunktionalen Griff begrenzter Hohlraum in dem multifunktionalen Griff an der Unterseite des multifunktionalen Griffs geschaffen. In diesem Hohlraum kann beispielsweise ein funktionelles Bauteil wie ein Lüftervlies angeordnet sein. Dies hat den Vorteil, dass der multifunktionale Griff das Lüftervlies von drei Seiten gegen ungewollte Berührungen mit den Fingern oder sonstige schädigende Einflüsse abschirmt. Zudem ist der Zugang zu dem Lüftervlies sehr verwinkelt, wodurch die Wahrscheinlichkeit beispielsweise einer ungewollten Berührung des Lüftervlieses weiter reduziert wird.

### Figurenbeschreibung

Die Erfindung wird nachstehend anhand bevorzugter Ausführungsbeispiele unter Bezugnahme auf die begleitenden Figuren näher erläutert.

### Hierbei zeigen

Fig. 1a bis 1f unterschiedliche Konstruktionsbeispiele eines erfindungsgemäßen multifunktionalen Griffs, jeweils mit einem bügel- oder rinnenartigen Leitungsaufnahmeabschnitt (nachfolgend in Auflagebereich/-abschnitt und Abrutschverhinderungsbereich/-abschnitt untergliedert) und einen ergonomisch geformten Griffabschnitt,
Fig. 2 eine Blutbehandlungsmaschine (nachfolgend grundsätzlich als Dialysemaschine bezeichnet), an der der erfindungsgemäße multifunktionale Griff über ein Verbindungs- oder Adapterstück montiert ist, und
Fig. 3 die Dialysemaschine gemäß der Fig. 2 mit dem erfindungsgemäßen multifunktionalen Griff, auf welchem Leitungen oder Schläuche aufgewickelt sind.
Fign.4a und b jeweils eine Detailansicht aus einem unterschiedlichen Blickwinkel eines erfindungsgemäßen multifunktionalen Griffs gemäß einer weiteren Ausführungsform der Erfindung, welcher an einer Dialysemaschine montiert ist und eine integrierte Medienleiste mit elektrischen und IT-Schnittstellen, sowie einen Hohlraum zur Abschirmung eines Lüftervlieses aufweist.

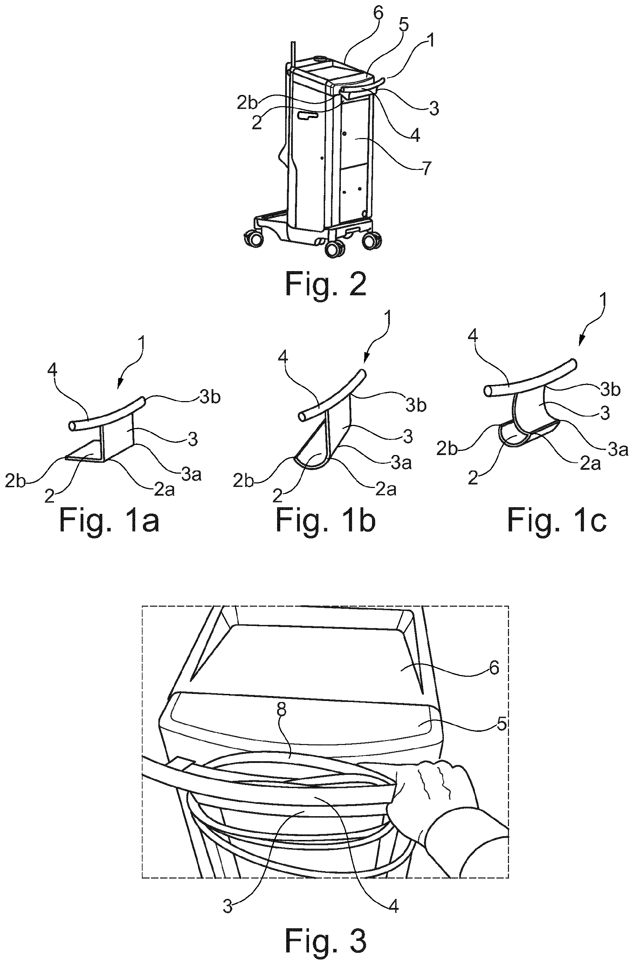

Fig. 1a zeigt die ersten Ausführungsform eines multifunktionalen Griffs 1 für eine Blutbehandlungs- bzw. Dialysemaschine zum händischen Bewegen der Dialysemaschine sowie zum Aufwickeln von Leitungen und/oder Schläuchen, mit einem flächigen, bzw. plattenförmigen Auflagebereich/-abschnitt 2 und einem flächigen bzw. plattenförmigen Abrutschverhinderungsbereich/-abschnitt 3, der an einem ersten kantenartigen Ende 2a des Auflageabschnitts 2 in einem Winkel von dem Auflageabschnitt 2 absteht. Die Abschnitte 2 und 3 bilden zusammen eine im Querschnitt V- oder L-förmige Rinne, welche im Zusammenwirken einen Leitungsaufnahmeabschnitt des Multifunktionshandgriffs definieren.

In dieser Ausführungsform ist der multifunktionale Griff 1 einstückig gefertigt und der Winkel zwischen dem Auflageabschnitt 2 und dem Abrutschverhinderungsabschnitt 3 ist im Wesentlichen rechtwinklig, kann aber auch ein spitzer oder stumpfer Winkel sein. Durch die im Wesentlichen rechtwinklige Anordnung entsteht der im Wesentlichen L-förmige Griff 1, bei dem die Fläche des Auflageabschnitts 2 die alleinige Auflage für Leitungen bildet, die um den Griff 1 aufgewickelt verwahrt werden können, wohingegen der Abrutschverhinderungsabschnitt 3 alleinig ein unbeabsichtigtes Abgleiten der bereits aufgewickelten Leitungen/Schläuche über die freie Kante des Auflageabschnitts 2 verhindern soll.

An seinem dem Auflageabschnitt 2 abgewandten, freien (kantenförmigen) Ende 3b weist der Abrutschverhinderungsabschnitt 3 zum verbesserten Greifen des Griffs 1 und zum vereinfachten Rangieren der mobilen Dialysemaschine, an der der multifunktionale Griff 1 fixiert ist, eine Griffstange bzw. einen Handlauf 4 auf, die bzw. der entlang der Kante des dem Auflageabschnitt 2 abgewandten Endes 3b des Abrutschverhinderungsabschnitts 3 verläuft. Die Griffstange 4 ragt hierbei an ihren beiden Enden um eine gleiche Länge über den Abrutschverhinderungsabschnitt 3 vor. Dies ermöglicht ein einfaches Rangieren der mobilen Dialysemaschine nach rechts und links und schützt zudem das Gehäuse der Dialysemaschine bei Kollisionen.

Die Oberfläche des gesamten multifunktionalen Griffs 1 besteht aus großen, spaltfreien und hinterschneidungsfreien Flächen mit abgerundeten Ecken. Dies vereinfacht die Reinigung des Griffs 1. Außerdem beugt es Beschädigungen der aufgewickelten Leitungen/Schläuchen etwa durch Knicken oder Eindrücken vor.

Der multifunktionale Griff 1 kann an dem zweiten (dem Abrutschverhinderungsabschnitt 3 abgewandten) Ende 2b des Auflageabschnitts 2 mit der Dialysemaschine, vorzugsweise über ein Verbindungs-/Adapterstück, verbunden werden. Bei dieser Anordnung liegt die durch den Auflageabschnitt 2 gebildete Auflagefläche für Leitungen zwischen der Oberfläche der Dialysemaschine und dem Abrutschverhinderungsabschnitt 3 mit dem daran angeordneten Handlauf/Griffstange 4. Da ein Benutzer den multifunktionalen Griff 1 an der Griffstange 4 greift, befindet sich die Auflagefläche daher, von dem Ergreifenden betrachtet, hinter der Griffstange 4 und etwaige auf der Auflagefläche befindliche Leitungen behindern somit ein Ergreifen des multifunktionalen Griffs 1 an der Griffstange 4 nicht.

Alternativ kann der multifunktionale Griff 1 auch an der durch den Abrutschverhinderungsabschnitt 3 gebildeten Fläche nach Art einer Rücken-an-Rücken-Verbindung mit der Dialysemaschine verbunden werden. Die durch den Auflageabschnitt 2 gebildete Auflagefläche befindet sich in dieser Anordnung, von dem Ergreifenden betrachtet, vor der Griffstange 4. Eine derartige Anordnung kann unter Umständen das Aufwickeln von Leitungen um den multifunktionalen Griff 1 erleichtern, da die Leitungen nicht um die Griffstange herumgeführt werden müssen. Jedoch wäre es in diesem Fall empfehlenswert, anstelle der vorstehend beschriebenen L-Form eine V- oder U-Form für den Leitungsaufnahmebereich zu wählen, um auch in diesem Fall ein unbeabsichtigtes Abrutschen der aufgewickelten Leitungen zu vermeiden.

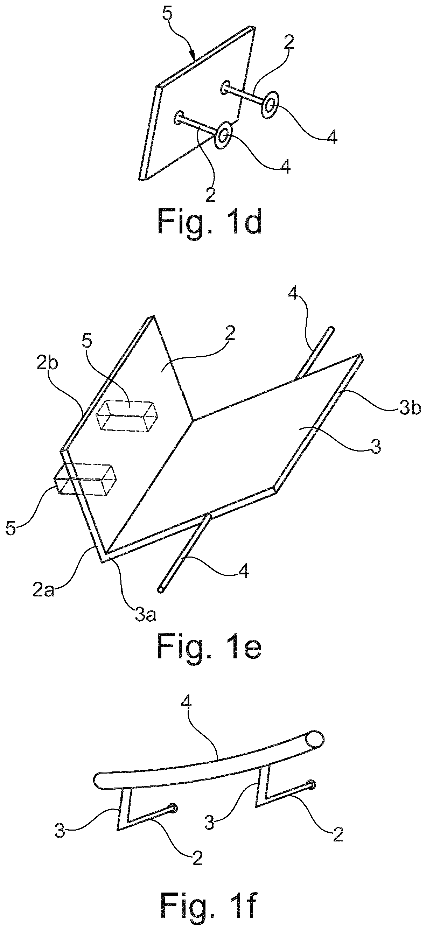

Der Abrutschverhinderungsabschnitt 3 kann gemäß der Fig. 1 a gerade oder gewölbt ausgebildet sein, so dass der Abrutschverhinderungsabschnitt 3 die Wölbung einer Oberfläche der Dialysemaschine, an der der multifunktionale Griff 1 montiert ist, nachbildet.

Fig. 1b zeigt einen erfindungsgemäßen multifunktionalen Griff 1, bei dem der Auflageabschnitt 2 per se (tonnenförmig) gerundet ausgebildet ist, so dass eine Aufnahmemulde/-rinne zur verbesserten Aufnahme von Leitungen gebildet wird. Dieselben Bezugszeichen, die auch in den übrigen Figuren verwendet werden, beziehen sich auf dieselben oder sehr ähnliche Bauteile.

Die gerundete Ausbildung des Auflageabschnitts 2 ermöglicht es, den multifunktionalen Griff 1 kompakter auszubilden, so dass der multifunktionale Griff 1 um eine geringere Distanz von der Dialysemaschine vorsteht, als ein vergleichbarer multifunktionaler Griff 1, bei dem der Auflageabschnitt 2 plan ausgebildet ist, wie in der Fig. 1a gezeigt wird. Zusätzlich kann der Abrutschverhinderungsabschnitt 3 gewölbt ausgebildet sein, so dass der Abrutschverhinderungsabschnitt 3 die Wölbung einer Oberfläche der Dialysemaschine, an der der multifunktionale Griff 1 montiert ist, nachbildet.

Fig. 1c zeigt eine weitere Ausgestaltung eines erfindungsgemäßen multifunktionalen Griffs 1, bei dem die Fläche des Abrutschverhinderungsabschnitts 3 hin zu der Fläche des Auflageabschnitts 2 (tonnenförmig) gerundet ist. Bei der in dieser Figur dargestellten Ausgestaltung ist die Fläche des Auflageabschnitts 2 ebenfalls wie in der Fig. 1b gerundet ausgebildet, so dass eine vertiefte Aufnahmemulde/-rinne entsteht. Alternativ kann die Fläche des Auflageabschnitts 2 aber auch plan sein (nicht weiter gezeigt). Durch die gerundete Ausgestaltung des Abrutschverhinderungsabschnitts 3 gemäß der Fig. 1c kann vor allem bei einer Anordnung des multifunktionalen Griffs 1, bei der der multifunktionale Griff 1 an dem zweiten, dem Abrutschverhinderungsabschnitt 3 abgewandten (kantenförmigen) Ende 2b des Auflageabschnitts 2 mit der Dialysemaschine verbunden ist, ein unerwünschtes Herabfallen von in der Auflagemulde befindlichen Leitungen verhindert werden, da die Leitungen zwischen der Wölbung des Abrutschverhinderungsabschnitts 3 und der Oberfläche der Dialysemaschine quasi eingeklemmt sind.

In der Fig. 1e ist eine alternative Ausführungsform zum Ausführungsbeispiel gemäß der Fig. 1a gezeigt. In diesem Fall ist die Griffstange 4 nicht unmittelbar am freien (kantenförmigen) Ende 3b des Leitungsaufnahmeabschnitts/-bereichs sondern in dessen Mittenabschnitt platziert. Die Griffstange 4 ist in zwei Teile gegliedert, die jeweils an einer in Auflage-/Aufwickelrichtung gesehenen Stirnkante des Leitungsaufnahmeabschnitts/-bereichs fixiert sind und so den Leitungsaufnahmeabschnitt/- bereich zwischen sich aufnehmen. Diese Variante hat den Vorteil, dass beispielsweise zwei unterschiedliche Leitungen/Schläuche eingehängt werden können, welche von der zweigeteilten Griffstange 4 voneinander separiert werden können.

An dieser Stelle sei darauf hingewiesen, dass der Leitungsaufnahmeabschnitt/- bereich nicht notwendiger Weise plattenförmig sondern auch stangenförmig sein kann, wie dies in den Fig. 1d und 1f gezeigt ist. In diesem Fall sind der Auflageabschnitt 2 und/oder der Abrutschverhinderungsabschnitt 3 aus zumindest einer Stange gebildet. In diesem Fall kann der Griff eine Griffstange 4 haben, wie diese in der Fig. 1f dargestellt ist oder es können ein oder zwei Griffkugeln/Knöpfe an den freien Stangenenden angeordnet sein, welche gleichzeitig auch den Abrutschverhinderungsabschnitt 3 definieren können. Diese Variante ist in der Fig. 1 d gezeigt.

Fig. 2 zeigt eine Blutbehandlungsmaschine/ (Hämo-) Dialysemaschine 6, an der ein multifunktionaler Griff 1 gemäß der vorliegenden Erfindung über ein Verbindungsstück 5 fixiert ist. Hierbei ist der multifunktionale Griff 1 mit dem zweiten, dem Abrutschverhinderungsabschnitt 3 abgewandten (kantenförmigen) Ende 2b des Auflageabschnitts 2 mit dem Verbindungsstück 5 verbunden, welches fest an der Dialysemaschine 6 angebracht ist/wird. Bei dieser Anordnung befindet sich die durch den Auflageabschnitt 2 gebildete Auflagefläche bzw. -rinne für Leitungen zwischen der Griffstange 4 bzw. dem Abrutschverhinderungsabschnitt 3 und der Oberfläche der Dialysemaschine 6. Der Griff entspricht somit dem bereits vorstehend beschriebenen Griff gemäß der Fig. 1 a.

In dieser Darstellung ist klar zu erkennen, wie der von der Oberfläche der Dialysemaschine 6 vorragende Auflageabschnitt 2 des multifunktionalen Griffs 1 die Flanke/Seitenwand 7 der Dialysemaschine 6 nach Art eines vorstehenden Daches vor von oben herabfallendem Spritzwasser schützt. Werden elektrische bzw. IT-Schnittstellen an der durch den hervorstehenden multifunktionalen Griff 1 derart geschützten Flanke 7 der Dialysemaschine 6 angeordnet, so entfällt das Erfordernis für separate Abdeckungen zum Schutz dieser Schnittstellen vor Spritzwasser.

In dieser Ausgestaltung ist die Baugruppe aus multifunktionalem Griff 1 und Verbindungsstück 5 stoffeinstückig gefertigt. Es kann aber auch sein, den Multifunktionsgriff 1 als Universalgriff zu konzipieren der dann mit unterschiedlichen Verbindungs-/Adapterstücken 5 kombinierbar ist, welche für unterschiedliche Blutbehandlungsmaschinen angepasst sind.

Fig. 3 zeigt eine Detailansicht eines multifunktionalen Griffs 1, der an einer Dialysemaschine 6 montiert ist. In dieser Darstellung sind von der Baugruppe aus multifunktionalem Griff 1 und Verbindungsstück 5 der Abrutschverhinderungsabschnitt 3 und die Griffstange 4 des multifunktionalen Griffs 1, sowie das Verbindungsstück 5 zu sehen. Der Auflageabschnitt 2 des multifunktionalen Griffs 1 liegt unter den auf den multifunktionalen Griff 1 aufgewickelten Leitungen 8 und ist daher in dieser Darstellung nicht sichtbar. Ein Benutzer ergreift die Griffstange 4 an ihrem rechten, über den Abrutschverhinderungsabschnitt 3 des multifunktionalen Griffs 1 hervorragenden Ende. Die Leitungen 8 liegen bei Betrachtung von dem den multifunktionalen Griff 1 Ergreifenden aus betrachtet, hinter der Griffstange 4 und behindern daher ein Ergreifen der Griffstange 4 nicht.

Der Abrutschverhinderungsabschnitt 3, sowie die Griffstange 4 sind gewölbt ausgebildet, so dass sie die Wölbung der Oberfläche der Dialysemaschine 6 bzw. des Verbindungsstücks 5 nachbilden. Der multifunktionale Griff 1 dient hierbei auch als Begrenzungselement für die Dialysemaschine 6, da er nach Art einer Stoßstange das Gehäuse der Dialysemaschine 6 bei einer Kollision mit Wänden oder ähnlichem schützt.

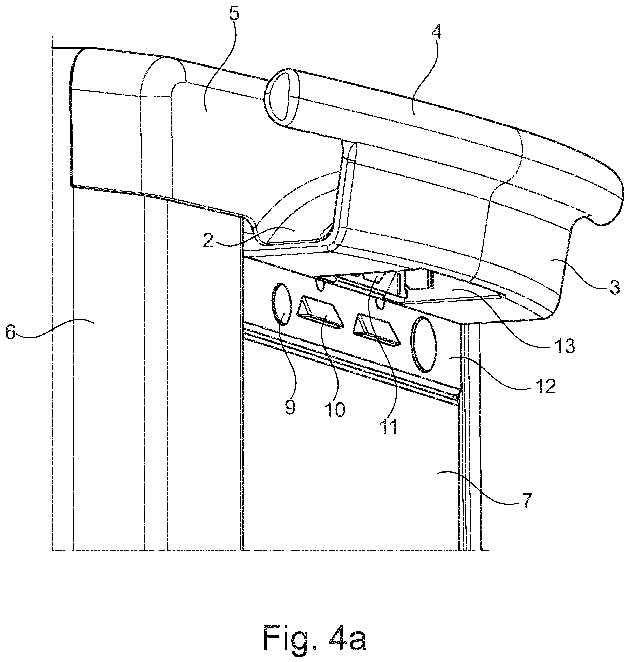

Fig.4a zeigt eine Detailansicht eines erfindungsgemäßen multifunktionalen Griffs 1 gemäß einer weiteren Ausführungsform der Erfindung, welcher an einer Blutbehandlungsmaschine bzw. Dialysemaschine 6 montiert ist und eine integrierte Medienleiste 12 mit elektrischen bzw. IT-Schnittstellen 9 aufweist. Zusätzlich weist der multifunktionale Griff auch Öffnungen 10 auf. Diese Öffnungen 10 dienen beispielsweise dazu, die Dämpfung des Signals eines in der Blutbehandlungsmaschine montierten Lautsprechers zu reduzieren, da durch die Öffnungen 10 der von dem Lautsprecher ausgesandte Schall aus der Blutbehandlungsmaschine nach außen dringen kann.

Die Medienleiste 12 ist an der durch den multifunktionalen Griff 1 vor von oben kommendem Spritzwasser geschützten Flanke 7 der Blutbehandlungsmaschine 6 bei Betrachtung von der Oberseite der Blutbehandlungsmaschine 6 her hinter /unter dem multifunktionalen Griff 1 angeordnet. Der Leitungsaufnahmeabschnitt 2 des multifunktionalen Griffs 1 ist in dieser Ausgestaltung als ein Ausschnitt einer Zylindermantelfläche ausgebildet, der hin zu der Oberseite der Blutbehandlungsmaschine 6 konvex gewölbt ist. Durch diese geometrische Ausgestaltung des Leitungsaufnahmeabschnitts 2 des multifunktionalen Griffs 1 wird ein Hohlraum 13 an der Unterseite des Leitungsaufnahmeabschnitts 2 des multifunktionalen Griffs 1 gebildet. In diesem Hohlraum 13 ist ein Lüftervlies 11, über welches das Gehäuseinnere mit der Atmosphäre verbunden ist, angeordnet. Da der Leitungsaufnahmeabschnitt 2 des multifunktionalen Griffs 1 den Hohlraum 13 an drei Seiten (Oberseite, linke Seite, rechte Seite) begrenzt, ist das Lüftervlies 11 an den drei Seiten vor unerwünschten Berührungen mit den Fingern, Spritzwasser und sonstigen schädigenden Einflüssen abgeschirmt. Durch die Ausgestaltung des multifunktionalen Griffs 1 bzw. durch die Anordnung des multifunktionalen Griffs 1 an der Dialysemaschine 6 ist der Zugang zu dem Lüftervlies 11 oder jedem anderen beliebigen in dem Hohlraum 13 angeordneten Element verwinkelt und somit sehr erschwert. Durch diese Anordnung läßt sich die Wahrscheinlichkeit unerwünschter Berührungen des Lüftervlieses 11 durch Bedienpersonal weiter reduzieren.

Der Zugang zu den an der integrierten Medienleiste 12 angeordneten elektrischen bzw. IT-Schnittstellen 9 ist auch dann gewährleistet, wenn Leitungen auf dem Leitungsaufnahmeabschnitt 2 des multifunktionalen Griffs 1 aufgewickelt sind. Hierfür ist der Leitungsaufnahmeabschnitt 2 des multifunktionalen Griffs 1 so in seinen Abmessungen eingestellt und die Anordnung der elektrischen bzw. und IT-Schnittstellen 9 an der integrierten Medienleiste 12 und der Öffnungen 10 ist derart, dass die auf dem Leitungsaufnahmeabschnitt 2 aufliegenden Leitungen 8 aufgrund der intrinsischen Steifigkeit der Leitungen 8 genügend von dem multifunktionalen Griff 1 abstehen, dass die Schnittstellen 9 und auch die Öffnungen 10 nicht von den Leitungen 8 verdeckt werden.

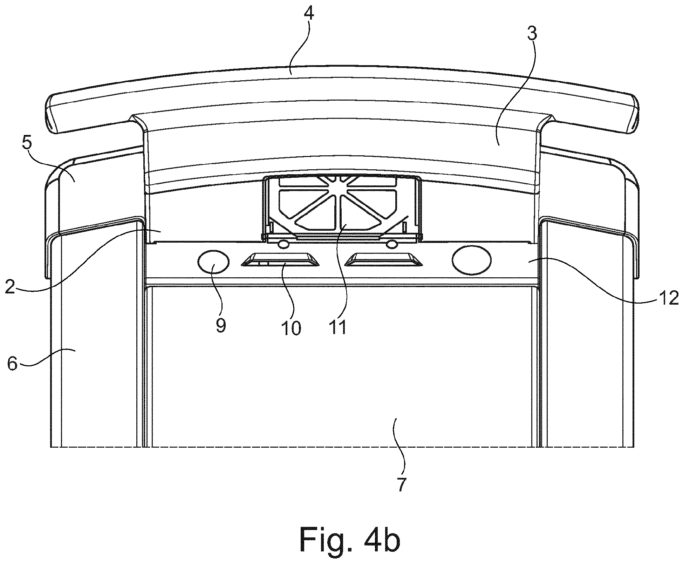

Fig.4b zeigt eine Frontalansicht des multifunktionalen Griffs 1 aus Fig. 4a. Demnach befindet sich das Lüftervlies 11 oberhalb der Medienleiste 12 und unterhalb des dachförmig gewölbten Leitungsaufnahmeabschnitts 2.

## Patentansprüche

1. Multifunktionaler Griff (1) einer mobilen Blutbehandlungsmaschine (6) zum händischen Bewegen der Blutbehandlungsmaschine (6) sowie zum Aufwickeln oder Einhängen von Leitungen und/oder Schläuchen (8), umfassend einen bügel- oder rinnenartigen Leitungsaufnahmeabschnitt (2) und einen ergonomisch geformten Griffabschnitt, der an wenigstens einem Endbereich des bügel- oder rinnenartigen Leitungsaufnahmeabschnitts angeordnet oder ausgebildet ist, und **gekennzeichnet dadurch, dass** der Leitungsaufnahmeabschnitt (2) einen Hohlraum (13) zur besonders abgeschirmten Unterbringung eines funktionellen Bauteils ausbildet.

2. Multifunktionaler Griff (1) einer mobilen Blutbehandlungsmaschine (6) gemäß Anspruch 1, mit einem Abrutschverhinderungsabschnitt (3), der zwischen dem bügel-oder rinnenartigen Leitungsaufnahmeabschnitt (2) und dem ergonomisch geformten Griffabschnitt (4) angeordnet und hin zu der mobilen Blutbehandlungsmaschine (6) derart gewölbt ist, dass ein Abrutschen von in dem bügel- oder rinnenartigen Leitungsaufnahmeabschnitt (2) aufgenommenen Leitungen und / oder Schläuchen (8) verhindert wird.

3. Multifunktionaler Griff (1) nach Anspruch 1 oder 2, **dadurch gekennzeichnet, dass** der Griffabschnitt handlauf- oder stangenartig ausgeformt ist und sich längs zu einer durch den Leitungsaufnahmeabschnitt definierten Leitungsaufnahmerichtung erstreckt.

4. Multifunktionaler Griff (1) nach Anspruch 3, **dadurch gekennzeichnet, dass** der Griffabschnitt an einem quer zur Leitungsaufnahmerichtung sich befindenden Ende des Leitungsaufnahmeabschnitts positioniert ist.

5. Multifunktionaler Griff (1) nach Anspruch 3, **dadurch gekennzeichnet, dass** der Griffabschnitt an einem längs zur Leitungsaufnahmerichtung sich befindenden Ende des Leitungsaufnahmeabschnitts positioniert ist.

6. Multifunktionaler Griff (1) nach einem der vorhergehenden Ansprüche, **dadurch gekennzeichnet, dass** der Leitungsaufnahmeabschnitt (2) des multifunktionalen Griffs (1) an einem dem Griffabschnitt gegenüberliegenden Anschlussende fest mit der mobilen Blutbehandlungsmaschine (6), vorzugsweise über ein Verbindungsstück oder Montageadapter (5), verbunden oder verbindbar ist.

7. Multifunktionaler Griff (1) nach einem der vorhergehenden Ansprüche, **dadurch gekennzeichnet, dass** der Leitungsaufnahmeabschnitt (2) des multifunktionalen Griffs (1) als Abstandshalter des Griffabschnitts zur Blutbehandlungsmaschine (6) dient.

8. Multifunktionaler Griff (1) nach einem der vorhergehenden Ansprüche, **dadurch gekennzeichnet, dass** der vorzugsweise handlauf- oder stangenartige Griffabschnitt (4) an mindestens einem und vorzugsweise beiden seiner Längsenden in Leitungsaufnahmerichtung gesehen über den Leitungsaufnahmeabschnitt (2) vorragt.

9. Multifunktionaler Griff (1) nach einem der vorhergehenden Ansprüche, **dadurch gekennzeichnet, dass** der Leitungsaufnahmeabschnitt (2) in Leitungsaufnahmerichtung gesehen schalenförmig gewölbt ausgebildet ist, um ein Abknicken der eingehängten Leitungen und/oder Schläuche zu vermeiden.

10. Multifunktionaler Griff (1) nach einem der vorhergehenden Ansprüche, **dadurch gekennzeichnet, dass** der Leitungsaufnahmeabschnitt (2) als flächiges, vorzugsweise plattenartiges Bauteil ausgebildet ist.

11. Multifunktionaler Griff (1) nach einem der vorhergehenden Ansprüche, **dadurch gekennzeichnet, dass** der multifunktionale Griff (1), vorzugsweise mit einem Verbindungsstück oder Montageadapter (5), stoffeinstückig aus Metall oder Kunststoff gefertigt ist.

12. Multifunktionaler Griff (1) nach einem der vorhergehenden Ansprüche, **dadurch gekennzeichnet, dass** in dem multifunktionalen Griff (1) elektrische und andere Schnittstellen (9), vorzugsweise in einer integrierten Medienleiste (12), integriert sind.

13. Multifunktionaler Griff (1) nach Anspruch 12, **dadurch gekennzeichnet, dass** der multifunktionale Griff (1) die elektrischen und anderen Schnittstellen (9) mindestens vor aus einer Richtung kommendem Spritzwasser abschirmt.

14. Mobile Blutbehandlungsmaschine (6) mit einem multifunktionalen Griff (1) nach einem der Ansprüche 1 bis 13.

15. Mobile Blutbehandlungsmaschine (6) mit einem multifunktionalen Griff (1) gemäß einem der Ansprüche 12 und 13, **dadurch gekennzeichnet, dass** der multifunktionale Griff (1) derart an der mobilen Blutbehandlungsmaschine (6) montiert ist, dass der multifunktionale Griff (1) die elektrischen und anderen Schnittstellen (9) von von der Oberseite der mobilen Blutbehandlungsmaschine (6) kommendem Spritzwasser abschirmt.

16. Mobile Blutbehandlungsmaschine (6) mit einem multifunktionalen Griff (1) gemäß einem der Ansprüche 1 bis 13, **dadurch gekennzeichnet, dass** der multifunktionale Griff (1) derart an der mobilen Blutbehandlungsmaschine (6) montiert ist, dass der multifunktionale Griff (1) einen definierten, seitlich abgeschirmten Zugang zu dem Hohlraum (13) ausbildet, der ein unbeabsichtigtes Berühren des funktionellen Bauteils erschwert.

## Claims

1. Multifunctional handle (1) of a mobile blood treatment machine (6) for manually moving the blood treatment machine (6) and for winding or suspending cables and/or tubes (8), comprising a bracket-like or groove-like cable receiving portion (2) and an ergonomically shaped handle portion that is arranged or formed at least at one end portion of the bracket-like or groove-like cable receiving portion, and **characterised in that** the cable receiving portion (2) forms a hollow space (13) for receiving a functional component in a particularly protected manner.

2. Multifunctional handle (1) of a mobile blood treatment machine (6) according to claim 1, having a slip prevention portion (3) that is arranged between the bracket-like or groove-like cable receiving portion (2) and the ergonomically shaped handle portion (4) and curved towards the mobile blood treatment machine (6) in such a way that slipping of cables and/or tubes (8) received in the bracket-like or groove-like cable receiving portion (2) is prevented.

3. Multifunctional handle (1) according to claim 1 or 2, **characterised in that** the handle portion is formed in a handrail-like or rod-like manner and extends along a cable receiving direction defined by the cable receiving portion.

4. Multifunctional handle (1) according to claim 3, **characterised in that** the handle portion is positioned at an end of the cable receiving portion that is transverse to the cable receiving direction.

5. Multifunctional handle (1) according to claim 3, **characterised in that** the handle portion is positioned at an end of the cable receiving portion that is along the cable receiving direction.

6. Multifunctional handle (1) according to any one of the preceding claims, **characterised in that** the cable receiving portion (2) of the multifunctional handle (1) is firmly connected or connectable to the mobile blood treatment machine (6) at a connection end opposite the handle portion, preferably via a connector or mounting adapter (5).

7. Multifunctional handle (1) according to any one of the preceding claims, **characterised in that** the cable receiving portion (2) of the multifunctional handle (1) serves as a spacer of the handle portion for the blood treatment machine (6).

8. Multifunctional handle (1) according to any one of the preceding claims, **characterised in that** the preferably handrail-like or rod-like handle portion (4) protrudes beyond the cable receiving portion (2) at least at one and preferably both of its longitudinal ends viewed in the cable receiving direction.

9. Multifunctional handle (1) according to any one of the preceding claims, **characterised in that** the cable receiving portion (2) is formed in a cup-shaped curved manner viewed in the cable receiving direction to prevent kinking of the suspended cables and/ortubes.

10. Multifunctional handle (1) according to any one of the preceding claims, **characterised in that** the cable receiving portion (2) is formed as a flat, preferably platelike component.

11. Multifunctional handle (1) according to any one of the preceding claims, **characterised in that** the multifunctional handle (1) is produced in one piece from metal or plastic, preferably with a connector or mounting adapter (5).

12. Multifunctional handle (1) according to any one of the preceding claims, **characterised in that** electrical and other interfaces (9) are integrated in the multifunctional handle (1), preferably in an integrated media strip (12).

13. Multifunctional handle (1) according to claim 12, **characterised in that** the multifunctional handle (1) shields the electrical and other interfaces (9) at least from splashing water coming from one direction.

14. Mobile blood treatment machine (6) with a multifunctional handle (1) according to any one of claims 1 to 13.

15. Mobile blood treatment machine (6) with a multifunctional handle (1) according to any one of claims 12 and 13,
**characterised in that** the multifunctional handle (1) is mounted on the mobile blood treatment machine (6) in such a way that the multifunctional handle (1) shields the electrical and other interfaces (9) from splashing water coming from the upper side of the mobile blood treatment machine (6).

16. Mobile blood treatment machine (6) with a multifunctional handle (1) according to any one of claims 1 to 13,
**characterised in that** the multifunctional handle (1) is mounted on the mobile blood treatment machine (6) in such a way that the multifunctional handle (1) forms a defined, laterally shielded access to the hollow space (13), which makes it difficult to inadvertently touch the functional component.

## Revendications

1. Poignée multifonctionnelle (1) d'une machine mobile de traitement du sang (6) destinée à déplacer manuellement la machine de traitement du sang (6) et à enrouler ou accrocher des conduits et/ou des tuyaux (8), comprenant une partie de logement de tuyaux (2) du type étrier ou gouttière et une partie formant poignée qui a une forme ergonomique et qui est conçue ou agencée au niveau d'au moins une zone terminale de la partie de logement de tuyaux du type étrier ou gouttière,
**caractérisée en ce que** la partie de logement de tuyaux (2) forme un espace creux (13) destiné à loger de manière particulièrement protégée un élément fonctionnel.

2. Poignée multifonctionnelle (1) d'une machine mobile de traitement du sang (6) selon la revendication 1, avec une partie antiglisse (3), qui est agencée entre la partie de logement de tuyaux (2) du type étrier ou gouttière et la partie formant poignée (4) ergonomique et qui est convexe par rapport à la machine mobile de traitement du sang (6) de manière à empêcher tout glissement de conduits et/ou tuyaux (8) logés dans la partie de logement de tuyaux (2) du type étrier ou gouttière.

3. Poignée multifonctionnelle (1) selon la revendication 1 ou 2, **caractérisée en ce que** la partie formant poignée est en forme de main courante ou de barre et s'étend le long d'une direction de logement de tuyaux définie par la partie de logement de tuyaux.

4. Poignée multifonctionnelle (1) selon la revendication 3, **caractérisée en ce que** la partie formant poignée est placée au niveau d'une extrémité, située transversalement par rapport à la direction de logement de tuyaux, de la partie de logement de tuyaux.

5. Poignée multifonctionnelle (1) selon la revendication 3, **caractérisée en ce que** la partie formant poignée est placée au niveau d'une extrémité, située longitudinalement par rapport à la direction de logement de tuyaux, de la partie de logement de tuyaux.

6. Poignée multifonctionnelle (1) selon l'une quelconque des revendications précédentes, **caractérisée en ce que**, au niveau d'une extrémité de raccordement située à l'opposé de la partie formant poignée, la partie de logement de tuyaux (2) de la poignée multifonctionnelle (1) est ou peut être reliée de manière fixe à la machine mobile de traitement du sang (6), de préférence par l'intermédiaire d'une pièce de liaison ou d'un adaptateur de montage (5).

7. Poignée multifonctionnelle (1) selon l'une quelconque des revendications précédentes, **caractérisée en ce que** la partie de logement de tuyaux (2) de la poignée multifonctionnelle (1) sert de pièce d'écartement de la partie formant poignée par rapport à la machine de traitement du sang (6).

8. Poignée multifonctionnelle (1) selon l'une quelconque des revendications précédentes, **caractérisée en ce que** la partie formant poignée (4) de préférence du type main courante ou barre dépasse, vue dans la direction de logement de tuyaux, au niveau d'au moins une de ses extrémités longitudinales et de préférence de ses deux extrémités longitudinales, au-delà de la partie de logement de tuyaux (2).

9. Poignée multifonctionnelle (1) selon l'une quelconque des revendications précédentes, **caractérisée en ce que** la partie de logement de tuyaux (2) est conçue convexe en forme de cuvette, vue dans la direction de logement de tuyaux, pour éviter une rupture par pliage des conduits et/ou tuyaux accrochés.

10. Poignée multifonctionnelle (1) selon l'une quelconque des revendications précédentes, **caractérisée en ce que** la partie de logement de tuyaux (2) est conçue comme un élément plat, de préférence du type plaque.

11. Poignée multifonctionnelle (1) selon l'une quelconque des revendications précédentes, **caractérisée en ce que** la poignée multifonctionnelle (1), de préférence avec une pièce de liaison ou un adaptateur de montage (5), est fabriquée dans un même matériau, en métal ou en plastique.

12. Poignée multifonctionnelle (1) selon l'une quelconque des revendications précédentes, **caractérisée en ce que** des interfaces électriques et autres (9), de préférence dans une barre de médias (12) intégrée, sont intégrées dans la poignée multifonctionnelle (1).

13. Poignée multifonctionnelle (1) selon la revendication 12, **caractérisée en ce que** la poignée multifonctionnelle (1) protège les interfaces électriques et autres (9) au moins contre de l'eau projetée à partir d'une direction.

14. Machine mobile de traitement du sang (6) avec une poignée multifonctionnelle (1) selon l'une quelconque des revendications 1 à 13.

15. Machine mobile de traitement du sang (6) avec une poignée multifonctionnelle (1) selon l'une des revendications 12 et 13, **caractérisée en ce que** la poignée multifonctionnelle (1) est montée de telle sorte sur la machine mobile de traitement du sang (6) que la poignée multifonctionnelle (1) protège les interfaces électriques et autres (9) contre de l'eau projetée à partir du dessus de la machine mobile de traitement du sang (6).

16. Machine mobile de traitement du sang (6) avec une poignée multifonctionnelle (1) selon l'une quelconque des revendications 1 à 13, **caractérisée en ce que** la poignée multifonctionnelle (1) est montée de telle sorte sur la machine mobile de traitement du sang (6) que la poignée multifonctionnelle (1) forme un tel accès défini et protégé latéralement à l'espace creux (13) qu'il rend plus difficile un contact involontaire avec l'élément fonctionnel.
